# EUROPEAN PATENT APPLICATION

(11) **EP 3 278 705 A1**
(43) Date of publication of application: **07.02.2018**
(21) Application number: 15887769.6
(22) Date of filing: 04.12.2015
(51) Int. Cl.: A61B 1/00, A61B 1/04, G02B 23/24

(54) **INSERTION DEVICE**

(30) Priority: 31.03.2015 JP 2015073634
(71) Applicant: OLYMPUS CORPORATION, Hachioji-shi Tokyo 192-8507 (JP)
(72) Inventor: UMEMOTO, Yoshitaka, Hachioji-shi, Tokyo 192-8507 (JP); YAMASHITA, Takashi, Hachioji-shi, Tokyo 192-8507 (JP); SUZUKI, Takashi, Hachioji-shi, Tokyo 192-8507 (JP); ONODA, Fumiyuki, Hachioji-shi, Tokyo 192-8507 (JP); ONDA, Takuro, Hachioji-shi, Tokyo 192-8507 (JP); OMOTO, Keijiro, Hachioji-shi, Tokyo 192-8507 (JP); NATORI, Yasuaki, Hachioji-shi, Tokyo 192-8507 (JP)
(74) Representative: Schicker, Silvia
(86) International application number: PCT/JP2015/084180
(87) International publication number: WO 2016/157627

(57) **Abstract**

An insertion apparatus includes an insertion section (110) configured to be freely bendable, a spiral tube (132) provided on an outer peripheral surface of the insertion section (110) to be rotatable about a longitudinal axis, a motor (150) that rotates the spiral tube (132), a drive control unit (202) that supplies a drive current to the motor (150) to control driving of the motor, a torque limit value storage unit (203) that stores a torque limit value which is an upper limit of the drive current, a drive current detection unit (2021) that detects the drive current, a bending shape detection unit (204) that detects a bending shape of the insertion section, and a torque limit value correction unit (206) that corrects the torque limit value stored in the torque limit value storage unit (203) in accordance with the bending shape detected by the bending shape detection unit (204).

## Description

### Technical Field

The present invention relates to a rotary self-propelled type insertion apparatus.

### Background Art

Generally, an insertion apparatus such as an endoscope apparatus is inserted into a lumen. Among these types of insertion apparatuses, an insertion apparatus, which is called a rotary self-propelled type, has been known. The rotary self-propelled type endoscope apparatus, for example, is provided with a rotating housing called a spiral tube, etc., in which a spiral-shaped fin is formed on an outer peripheral surface of the insertion section. When the rotating housing is rotated, the fin formed to the rotating housing is brought into contact with an inner wall of a lumen, and generates stress. The insertion section self-propels in an insertion direction or in a removal direction by the stress. A proposal regarding this type of insertion apparatus is disclosed, for example, in Jpn. Pat. Appln. KOKAI Publication No. 2014-064686.

### Summary of Invention

A general insertion apparatus has a torque limit function that stops rotation of a motor when a drive current of the motor for rotating a rotating housing reaches or exceeds a threshold. The general insertion apparatus is provided with, for example, a bending section configured to be freely bendable along a wall surface of a lumen. When a bending amount of the bending section in the rotating housing increases, a drive current necessary to rotate the rotating housing increases. In this case, the torque limit function is activated with a smaller load than with an intended load with which the torque limit function should be activated.

The present invention has been made in consideration of the above circumstances, and an object is to provide an insertion apparatus capable of activating the torque limit function when a certain load is applied regardless of a bending shape of the bending section.

To achieve the object, an insertion apparatus according to an aspect of the invention comprises: an insertion section configured to be freely bendable; a rotational housing provided on an outer peripheral surface of the insertion section to be rotatable about a longitudinal axis; a motor that rotates the rotational housing; a drive control unit that supplies a drive current to the motor to control driving of the motor; a torque limit value storage unit that stores a torque limit value which is an upper limit of the drive current; a drive current detection unit that detect the drive current; and a torque limit value correction unit that corrects the torque limit value stored in the torque limit value storage unit in accordance with a bending shape detected by a bending shape detection unit that detects a bending shape of the insertion section.

### Brief Description of Drawings

FIG. 1 is a schematic view of a configuration of an endoscope system as an example of an insertion apparatus according to an embodiment of the present invention.
FIG. 2A shows a bending shape in a basic state.
FIG. 2B shows a bending shape in a multi-bending state.
FIG. 3A shows an example of a torque limit setting table in a basic state.
FIG. 3B shows an example of a torque limit setting table in a multi-bending state.
FIG. 4 is a flowchart showing rotational control.
FIG. 5 is a flowchart showing torque limit control.
FIG. 6 is a flowchart showing correction control for a torque limit value.
FIG. 7A shows a U-shaped insertion section.
FIG. 7B shows a loop-shaped insertion section.
FIG. 8 shows a torque limit setting table in consideration of a bending shape of a section with no spiral tube.
FIG. 9 shows an example of a linear approximate equation of a curvature radius and a torque limit value.
FIG. 10 shows an example of a configuration of an endoscope system 1 in which a UPD probe as a shape detection sensor and a shape recognition unit are combined to detect a bending shape of an insertion section.

### Description of Embodiments

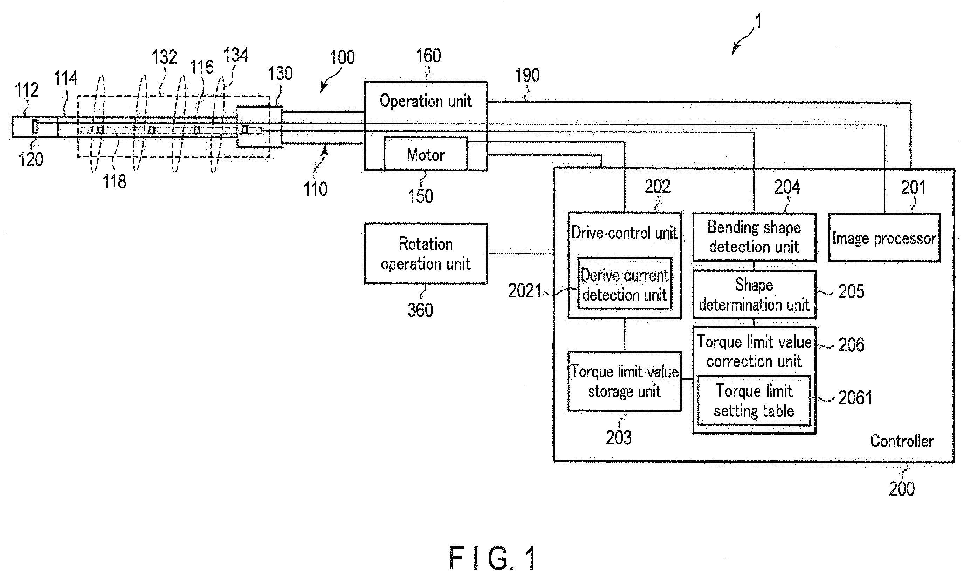

An embodiment of the present invention will be described with reference to the drawings. FIG. 1 is a schematic view of a configuration of an endoscope system as an example of an insertion apparatus according to an embodiment of the present invention. As illustrated in FIG. 1, the endoscope system 1 includes an endoscope 100, a controller 200, and a rotation operation unit 360.

The endoscope 100 is a rotary self-propelled type endoscope, and is provided with an insertion section 110. The insertion section 110 is an elongated shape, and is configured to be inserted in vivo. The endoscope 100 is attached to the insertion section 110 and provided with an operation unit 160 by which various operations for the endoscope 100 are performed. The operation unit 160 is held by a user. The operation unit 160 and the controller 200 of the endoscope 100 are connected by a universal cable 190. Hereinafter, the side of a distal end of the insertion section 110 is referred to as a distal end side. The side of the insertion section 110 in which the operation unit 160 is provided is referred to as a proximal end side. The direction from the distal end and the proximal end of the insertion section 110 is referred to as a longitudinal direction.

The insertion section 110 includes a distal end hard section 112, a bending section 114, and a coiled hose section 116. The distal end hard section 112 is an edge of the distal end of the insertion section 110, and is formed to not be bent.

The distal end hard section 112 includes an imaging element 120. The imaging element 120 generates an image signal based on a subject image at the distal end side of the insertion section 110, for example. The image signal generated by the imaging element 120 is transmitted to an image processor 201 of the controller 200 via an image signal line passing through the insertion section 110 and the universal cable 190. The bending section 114 is a section formed at the proximal end side of the distal end hard section 112, and is formed to be actively bent in accordance with an operation of an operating member (not shown) provided to the operation unit 160. The coiled hose section 116 is a section formed at the proximal end side of the bending section 114, and is formed to be passively bent by external force.

The coiled hose section 116 is provided with a rotation section 130. The rotating section 130 is provided with a spiral tube 132 which is a rotational housing at the distal end. The rotation section 130 transfers driving power of the motor 150 installed in the operation unit 160 to the spiral tube 132. The spiral tube 132 is made of a soft material such as rubber and resin to have a tubular shape. The spiral tube 132 is provided with a spiral fin 134 along the longitudinal axis of the spiral tube 132 on the outer peripheral surface. The spiral tube 132 may be configured to be removable from the rotation section 130.

A shape detection sensor 118 is provided inside the insertion section 110. The shape detection sensor 118 is a sensor for detecting a bending shape of the spiral tube 132 attached to the insertion section 110, and is arranged to detect a bending shape of a section, preferably multiple sections, of a portion provided with the at least spiral tube 132 in the insertion section 110. The shape detection sensor 118 may be arranged to detect a bending shape of the entire insertion section 110. The output signal of the shape detection sensor 118 is transmitted to a bending shape detection unit 204 of the controller 200 via a sensor signal line passing through the insertion section 110 and the universal cable 190. Examples of the shape detection sensor 118 include a distortion sensor that outputs a signal in accordance with bending (distortion) of the insertion section 110, a UPD (insertion shape observation apparatus) that detects the shape by detecting a magnetic field from a coil installed in the insertion section, and a sensor that detects the shape by the amount of light incident to a light sensor installed in the insertion section. The shape detection sensor 118 of the present embodiment is not limited to a sensor having the specified configuration, as long as the shape of the insertion section 110 can be detected.

The rotation section 130 is connected to the motor 150. The motor 150 is connected to a drive control unit 202 of the controller 200 via an actuator current signal line passing through the control unit 160 and the universal cable 190.

The motor 150 operates by an operation using a rotation operation unit 360. The rotative power of the motor 150 is transferred to the rotation section 130. As a result, the fin 134 provided to the spiral tube 132 is rotated around the longitudinal axis. If the fin 134 rotates in the state of being in contact with a wall part such as an inner wall of a lumen, stress is generated to allow the insertion section 110 to self-propel. For example, in a small intestine or a large intestine, the fin 134 draws folds in the inner wall of the small intestine or the large intestine so that stress is applied to the insertion section 110. The insertion section 110 self-propels by the stress. The self-propelling of the insertion section 110 assists the insertion operation or the removal operation of the insertion section 110 by the user. The motor 150 includes a pulse generator. The pulse generator generates a pulse signal (rotational speed signal) in accordance with the rotational speed of the motor 150, and inputs the rotational speed signal to the controller 200 via a signal line for rotational speed passing through the universal cable 190. The rotational speed of the motor 150 is controlled by the rotational speed signal.

The rotation operation unit 360 is, for example, a foot switch. The foot switch includes a pedal stepped on by the user and provides a command signal corresponding to a force of stepping on the pedal.

The controller 200 controls each element of the endoscope system 1. The controller 200 includes an image processor 201, a drive control unit 202, a torque limit value storage unit 203, a bending shape detection unit 204, a shape determination unit 205, and a torque limit value correction unit 206.

The image processor 201 performs image processing to an image signal input to the image processor 201 through the insertion section 110 and the universal cable 190. The image processor 201 inputs the processed image signal to a display unit (not shown) to display an endoscope image on the display unit.

The drive control unit 202 is made of ASIC, for example, and receives the command signal generated by the rotation operation unit 360 to obtain a command value. The drive control unit 202 obtains a rotational speed signal of the motor 150. The drive control unit 202 generates a drive current based on the command value and the rotational speed signal. The drive current is supplied to the motor 150 to drive the motor 150. Moreover, the drive control unit 202 includes a drive current detection unit 2021, and compares the drive current value and the torque limit value stored in the torque limit value storage unit 203, thereby stopping the rotation of the motor 150 when the drive current reaches or exceeds the torque limit value. This torque limit function can prevent generation of an excessive torque of the motor 150. Thus, it is possible to reduce unnecessary force applied to an inner wall of a lumen.

The torque limit value storage unit 203 stores a torque limit value that is the threshold for activating the torque limit function and is the upper limit value of the drive current. The torque limit value storage unit 203 is made of a nonvolatile memory, for example.

The bending shape detection unit 204 detects the bending shape of the spiral tube 132 from the output of the shape detection sensor 118. The bending shape is represented by a curvature radius [mm], for example. In this case, the bending shape detection unit 204 calculates the curvature radius from the output of the shape detection sensor 118.

The shape determination unit 205 determines to which of the predetermined characteristic bending states of the spiral tube 132 the bending shape detected by the bending shape detection unit 204 corresponds. Then, the shape determination unit 205 inputs a signal indicating the determination result to the torque limit value correction unit 206 with information on the curvature radius as the bending shape of the spiral tube 132. The predetermined bending states are, for example, two states of (1) basic state and (2) multi-bending state.

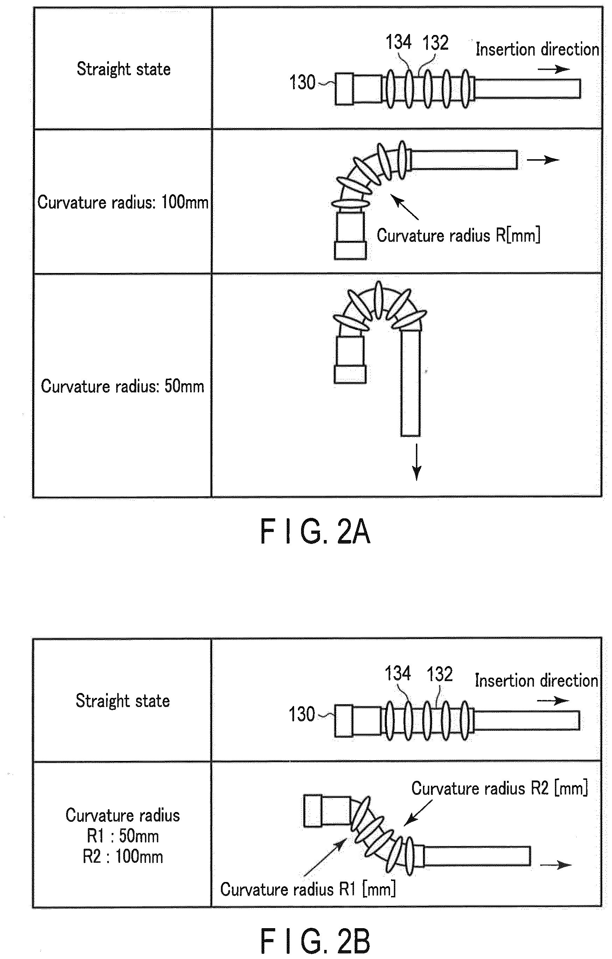

The basic state is a state in which one portion of the spiral tube 132 is bent as shown in FIG. 2A with respect to the straight state in which the spiral tube 132 has no bending portion. FIG. 2A shows an example in which the spiral tube 132 is bent at curvature radius R=100 [mm], and an example in which the spiral tube 132 is bent at curvature radius R=50 [mm].

The multi-bending state is a state in which multiple portions of the spiral tube 132 are bent in different directions as shown in FIG. 2B with respect to the straight state. FIG. 2B shows an example in which one portion at the proximal end side of the spiral tube 132 is bent at curvature radius R1=50 [mm] to be convex upward and one portion at the distal end side is bent at curvature radius R2=100 [mm] to be convex downward. The spiral tube 132 may be bent in an up-down direction and a right-left direction. When the spiral tube 132 is bent in both up-down and right-left directions, if one portion is curved in each direction, it is still regarded as the multi-bending state.

The torque limit value correction unit 206 includes a torque limit setting table 2061 in which the torque limit value in accordance with the bending shape is stored, and applies a correction by obtaining a torque limit value Ith_t corresponding to the bending shape determined by the shape determination unit 205 to rewrite the setting of the torque limit value storage unit 203.

In general, the hardness of the insertion section of the endoscope increases by bending. When a portion to which the spiral tube is attached in the insertion section becomes harder, the torque of the motor necessary to rotate the spiral tube increases. When the torque is equal to or greater than the torque limit value, the spiral tube cannot be rotated. In the present embodiment, correction is applied so that the torque limit value increases in accordance with an increase in the amount of bending, i.e., a reduction in the curvature radius. The torque limit setting table 2061 is a table in which the relationship between the curvature radius and the torque limit value is stored. The torque limit setting table is provided for each of the basic state and the multi-bending state.

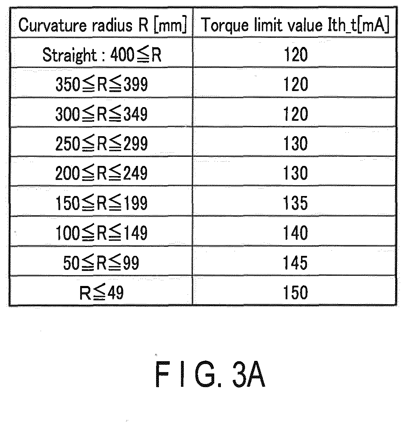

FIG. 3A shows an example of the torque limit setting table of the basic state. In the torque limit setting table of the basic state, when the curvature radius R is 400 [mm] or more, the effect on the torque limit function by bending is regarded as being equivalent to that in the straight state. With the curvature radius R=400 [mm] being used as a reference, when the curvature radius R is smaller than this, the torque limit value Ith_t becomes sequentially smaller accordingly. The actual value of the torque limit value Ith_t is obtained by, for example, an experiment or numerical analysis, and the values are shown in FIG. 3A by way of example.

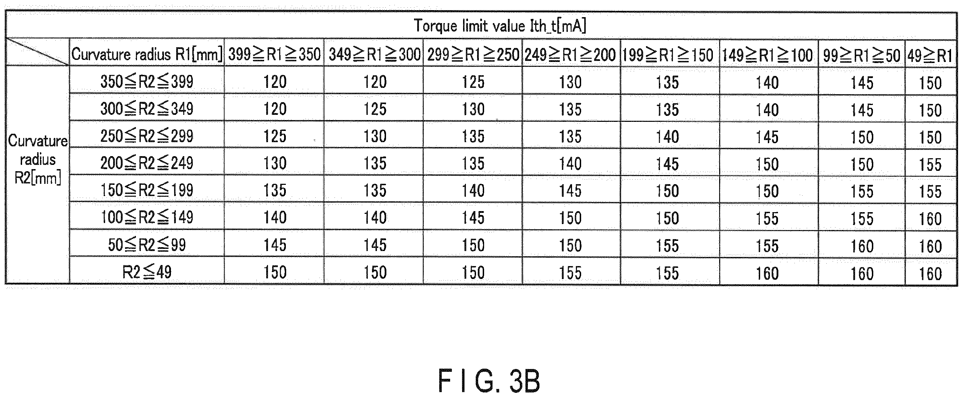

FIG. 3B shows an example of the torque limit setting table of the multi-bending state. As for the torque limit setting table of the multi-bending state, in principle, with the curvature radius R=400 [mm] being used as a reference, when the curvature radius R is smaller than this, the torque limit value becomes smaller. It should be noted that in the multi-bending state, a final torque limit value Ith_t is determined by a combination of two curvature radii R1 and R2. The actual value of the torque limit value Ith_t is obtained by, for example, an experiment or numerical analysis, and the values are shown in FIG. 3B by way of example. The multi-bending state may be a state in which three or more portions are bent. However, since the length of the spiral tube is imitative (approximately 300 [mm] at most), it is not likely that three or more portions are bent, which is more difficult than a case shown in FIG. 3B. Thus, settings of the torque limit setting table as illustrated in FIG. 3B are efficient.

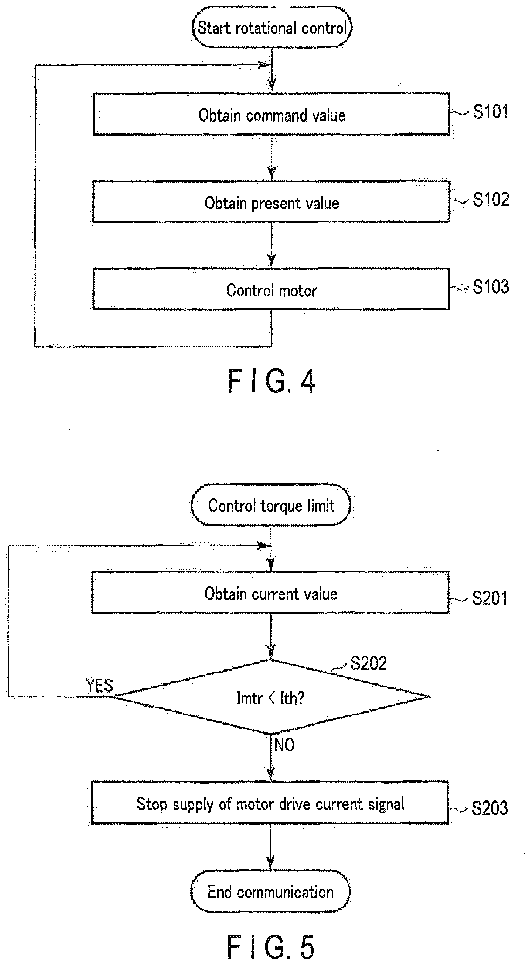

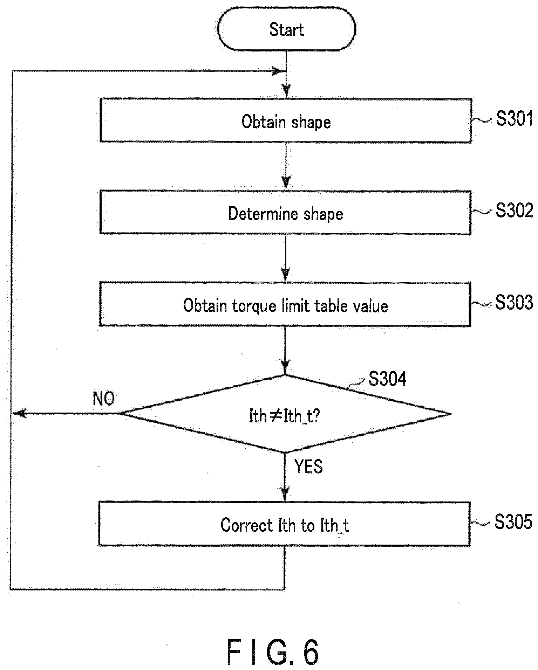

Hereinafter, a description will be given of operations of the endoscope system 1 according to the present embodiment. FIG. 4 is a flowchart showing rotational control of the motor 150 of the endoscope 100. FIG. 5 is a flowchart showing torque limit control. FIG. 6 is a flowchart showing correction control of a torque limit value. The controls of FIGS. 4, 5, and 6 are carried out in an asynchronous manner, for example. Of course, the controls of FIGS. 4, 5, and 6 may be carried out in a synchronous manner.

First, the rotational control will be described with reference to FIG. 4. The processing of FIG. 4 is carried out by the drive control unit 202. For example, the processing of FIG. 4 starts when the endoscope system 1 is powered on. In step S101, the drive control unit 202 receives a command signal from the rotation operation unit 360, and based on the command signal, obtains a command value of a rotational speed corresponding to the force of stepping on a pedal of the rotation operation unit 360 by the operator.

In step S102, the drive control unit 202 receives a rotational speed signal from the pulse generator of the motor 150, and based on this rotational speed signal, obtains a present value of the rotational speed.

In step S103, the drive control unit 202 generates a drive current signal corresponding to a deviation between the command value and the present value. The drive control unit 202 supplies the generated drive current signal to the motor 150 to drive the motor 150. Subsequently, the processing returns to step S101.

Next, the torque limit control will be described with reference to FIG. 5. The processing of FIG. 5 is carried out by the drive control unit 202. For example, the processing of FIG. 5 starts when the rotational control of FIG. 4 starts. In step S201, the drive control unit 202 obtains a present drive current value Imtr based on the present drive current signal.

In step S202, the drive control unit 202 compares the drive current value Imtr and the torque limit value Ith currently stored in the torque limit value storage unit 203 and determines whether Imtr is smaller than Ith (Imtr < Ith). When it is determined in step S202 that Imtr is smaller than Ith, the processing returns to step S201. When it is determined in step S202 that Imtr is not smaller than Ith, the processing advances to step S203.

In step S203, the drive control unit 202 stops supply of the drive current signal to the motor 150 to activate the torque limit function. Then, the processing is brought to an end. After the torque limit function is activated, once the pedal stepping operation is released, for example, the torque limit function is cancelled. When the torque limit function is cancelled, the processing of FIG. 5 resumes.

Next, the torque limit correction control will be described with reference to FIG. 6. The processing of FIG. 6 is performed by the bending shape detection unit 204, the shape determination unit 205, and the torque limit value correction unit 206. For example, the processing of FIG. 6 starts when the endoscope system 1 is powered on. In step S301, the bending shape detection unit 204 detects the bending shape of the insertion section 110 from the output of the shape detection sensor 118. Assuming that the shape detection sensor 118 is provided to detect a shape of multiple portions at the spiral tube 132, the bending shape detection unit 204 calculates each curvature radius of the portions as the bending shape of the spiral tube 132.

In step S302, the shape determination unit 205 determines to which of the basic state and the multi-bending state the bending shape detected by the bending shape detection unit 204 corresponds, and inputs the signal indicating the determination result to the torque limit value correction unit 206 along with information on the curvature radius as the bending shape of the spiral tube 132. Shape determination is made by determining whether the curvature radius of multiple portions is less than 400 mm, which indicates the straight state. When the curvature radius of the multiple portions is less than 400 mm, which indicates the straight state, it is determined to be a multi-bending state.

In step S303, the torque limit value correction unit 206 selects, in accordance with the determination result of the bending shape, either the torque limit setting table of the basic state illustrated in FIG. 3A, or the torque limit setting table of the multi-bending state illustrated in FIG. 3B. The torque limit value correction unit 206 obtains, from the selected torque limit setting table, a torque limit value Ith_t corresponding to the previously calculated curvature radius.

In step S304, the torque limit value correction unit 206 determines whether or not the torque limit value Ith stored in the torque limit value storing unit 203 and the torque limit value ith_t obtained in step S303 are different. When it is determined in step S304 that the torque limit value Ith and the torque limit value Ith_t are not different, the processing returns to step S301. In this case, the torque limit control of FIG. 5 is carried out by the torque limit value Ith previously stored in the torque limit storing unit 203. When it is determined in step S304 that the torque limit value Ith and the torque limit value Ith_t are different, the processing advances to step S305. In step S305, the torque limit value correction unit 206 applies a correction to replace the torque limit value Ith with the torque limit value Ith_t in step S304. Subsequently, the processing returns to step S301. In this case, the torque limit control illustrated in FIG. 5 is carried out by the replaced torque limit value Ith.

As described above, according to the present embodiment, the torque limit value is corrected in accordance with the bending shape of the spiral tube 132, and even when the bending shape of the spiral tube 132 changes, the torque limit function can be activated in the state in which the load actually applied to the spiral tube 132 is the same.

### [Modification 1]

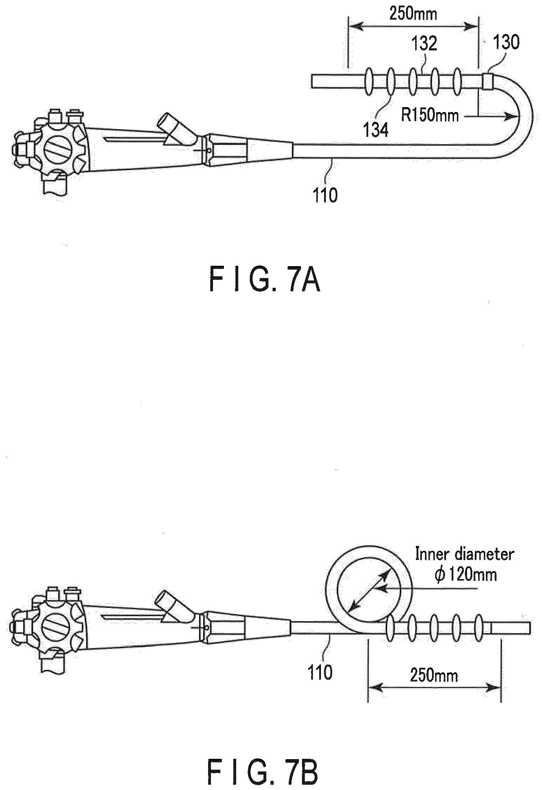

A description will be given of modifications of the present embodiment. The torque limit value correction unit 206 of the above-described embodiment corrects the torque limit value in accordance with the bending shape of the spiral tube 132. In addition, the torque limit value correction unit 206 may be configured to correct the torque limit value in accordance with the bending shape of a portion not provided with the spiral tube 132. For example, when the distal end of the insertion section 110 is U-shaped as illustrated in FIG. 7A or there is a loop in the middle of the insertion section 110 as illustrated in FIG. 7B, the rotation of the motor 150 is affected by a portion that is not located in the fin 134. Thus, it is more preferable to correct the torque limit value in consideration of the shape of the insertion section 110.

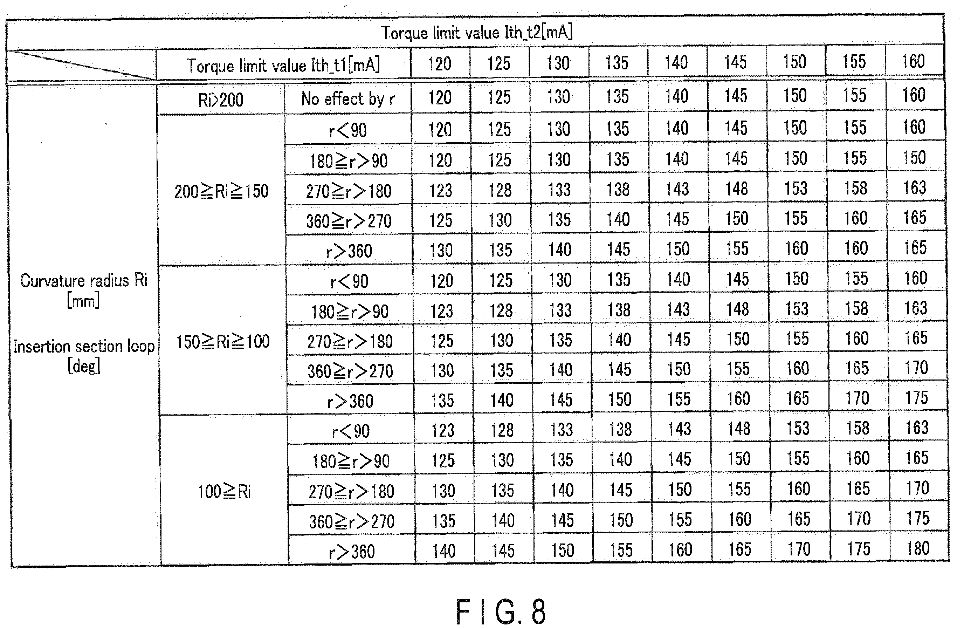

FIG. 8 shows a torque limit setting table in consideration of the bending shape of the portion not provided with the spiral tube 132. The torque limit setting table is a table for further correcting a torque limit value Ith_t1 (the above-described torque limit value Ith_t) obtained by using the table of FIG. 3A or FIG. 3B. In the table, the bending shape of the insertion section 110 is defined by, for example, a curvature radius Ri [mm] and a loop angle r [deg] . The loop angle r is an angle around a center point of a circular arc in which the curved portion of the insertion section 110 is a circumference.

In the example of FIG. 8, when the curvature radius Ri in the bending shape of the insertion section 110 is Ri>200 [mm], i.e., bending is not that much larger, there is no effect by the loop angle r. In contrast, when Ri is 200 [mm] or less, the value of torque limit value Ith_t2 becomes smaller as R1 is smaller and the loop angle r increases. The actual value of the torque limit value Ith_t2 is obtained by, for example, an experiment or numerical analysis, and the values are shown in FIG. 8 by way of example. For processing except for further correcting the torque limit value using the torque limit setting table of FIG. 8, the techniques of the above-described embodiment apply. Thus, detailed explanations will be omitted.

According to Modification 1 as described above, the torque limit value is corrected in accordance with the bending shape of the insertion section 110 of the portion other than the spiral tube 132, and more accurate torque limit control can be carried out.

### [Modification 2]

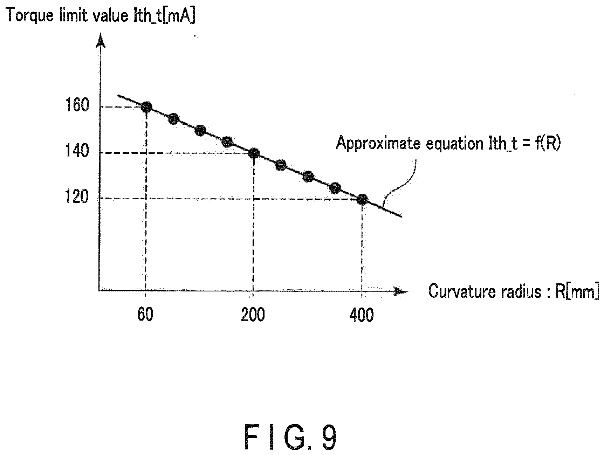

In the above-described embodiment and modification, the torque limit value is corrected with reference to the table. However, correcting the torque limit value does not necessarily have to be carried out with reference to the table. For example, the torque limit value correction unit 206 may be configured so that when the curvature radius R and the torque limit value Ith_t can be approximated by a linear equation Ith_t = f(R) (where f is a linear function) as shown in FIG. 9, correction is made using the linear equation instead of tables. FIG. 9 shows an example of a straight-line approximation, but the same applies to a case of curve approximation.

### [Modification 3]

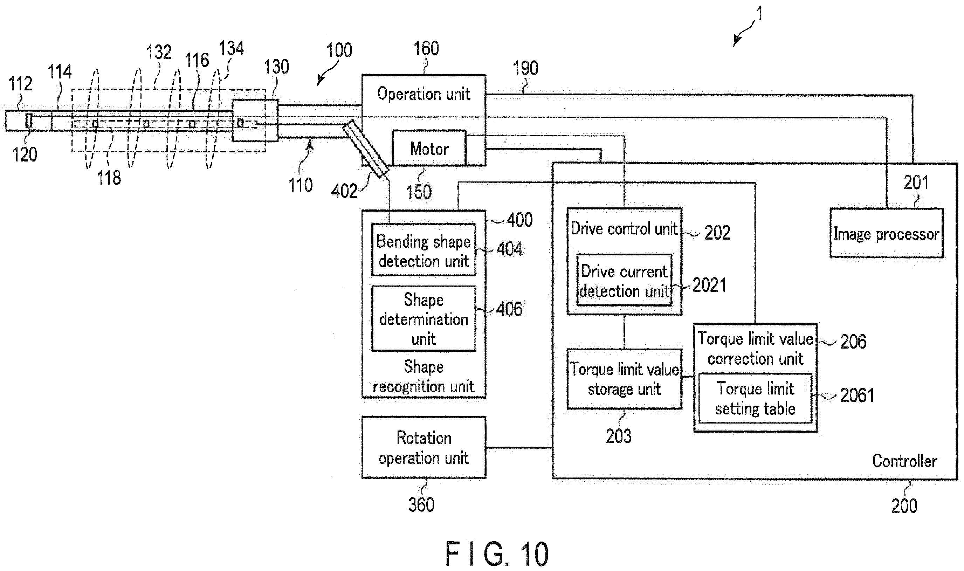

In the above-described embodiment and modifications, the bending shape detection unit and the shape determination unit are provided inside the controller 200. However, the bending shape detection unit and the shape determination unit may be provided outside the controller 200. Furthermore, the shape detection sensor may or may not be provided inside the insertion section 110. FIG. 10 shows an example of the configuration of the endoscope system 1 in which a UPD probe as a shape detection sensor 118 and a shape recognition unit 400 are combined to detect a bending shape of the insertion section 110.

The UPD probe as the shape detection sensor 118 is attached to the shape recognition unit 400. The UPD probe is inserted inside the insertion section 110 via a treatment instrument outlet 402 provided to the operation unit 160, for example.

The shape recognition unit 400 includes a bending shape detection unit 404 and a shape determination unit 406. The bending shape detection unit 404 has a configuration similar to that of the bending shape detection unit 204, and detects the bending shape of the spiral tube 132 from the output of the UPD probe. The shape determination unit 406 has a configuration similar to that of the shape determination unit 205 and determines to which of predetermined characteristic bending states of the spiral tube 132 the bending shape detected by the bending shape detection unit 404 corresponds. The shape determination unit 406 inputs the signal indicating the determination result to the torque limit value correction unit 206 with information on the curvature radius as the bending shape of the spiral tube 132 by communication between the controller 200 and the shape recognition unit 400. The torque limit value correction unit 206 applies a correction to rewrite the setting of the torque limit value storage unit 203 in accordance with the bending shape determined by the shape determination unit 406.

With the configuration as illustrated in FIG. 10, the bending shape detection unit and the shape determination unit can be separate from the controller 200. The shape detection sensor can be separate from the endoscope 100.

The present invention has been described based on the embodiments, but the present invention is in no way limited to those embodiments. The present invention can, of course, be modified in various manners, without departing from the spirit and scope of the inventions described in relation to the above embodiments may be stored in the form of programs executable by a CPU or the like as a computer. The programs can be stored in storage mediums of external storage devices, such as a memory card, a magnetic disk, an optical disk or a semiconductor memory, and distributed. The CPU or the like reads the programs from a storage medium of an external storage device, and the operations can be executed and controlled based on the read programs.

## Claims

1. An insertion apparatus comprising:
an insertion section configured to be freely bendable;
a rotational housing provided on an outer peripheral surface of the insertion section to be rotatable about a longitudinal axis;
a motor that rotates the rotational housing;
a drive control unit that supplies a drive current to the motor to control driving of the motor;
a torque limit value storage unit that stores a torque limit value which is an upper limit of the drive current;
a drive current detection unit that detect the drive current; and
a torque limit value correction unit that corrects the torque limit value stored in the torque limit value storage unit in accordance with a bending shape detected by a bending shape detection unit that detects a bending shape of the insertion section.

2. The insertion apparatus according to claim 1, further comprising:
a torque limit value correction table that stores the torque limit value in accordance with the bending shape,
wherein the torque limit value correction unit corrects the torque limit value stored in the torque limit value storage unit by obtaining a torque limit value corresponding to the bending shape detected by the bending shape detection unit.

3. The insertion apparatus according to claim 1,
wherein the drive control unit stops rotation of the motor when the drive current reaches or exceeds the torque limit value.

4. The insertion apparatus according to claim 1,
wherein the bending shape detection unit detects a bending shape of a portion provided with the rotational housing in the insertion section.

5. The insertion apparatus according to claim 1,
wherein the bending shape is represented by a curvature radius.

6. The insertion apparatus according to claim 1,
wherein the bending shape detection unit is provided in the insertion apparatus.

7. The insertion apparatus according to claim 1,
wherein the bending shape detection unit is provided outside the insertion apparatus.
